(19) Europäisches Patentamt
European Patent Office
Office européen des brevets

(11) Veröffentlichungsnummer: **0 027 891**
**B1**

(12) # EUROPÄISCHE PATENTSCHRIFT

(45) Veröffentlichungstag der Patentschrift: **09.03.83**

(21) Anmeldenummer: **80105672.2**

(22) Anmeldetag: **20.09.80**

(51) Int. Cl.³: **C 07 C 51/41**, C 07 C 57/32, C 07 C 59/68, C 07 C 63/08, C 07 C 79/38, C 07 C 79/44, C 07 C 85/00, C 07 C121/75, C 07 D215/12, C 07 D215/54, C 08 G 18/18

(54) **Ammoniumsalze, ein Verfahren zur Herstellung der ihnen zugrundeliegenden Amine, sowie die Ammoniumsalze enthaltende Beschichtungsmassen auf Basis von Polyurethan- oder Epoxiharzvorprodukten.**

(30) Priorität: **04.10.79 DE 2940333**

(43) Veröffentlichungstag der Anmeldung: **06.05.81 Patentblatt 81/18**

(45) Bekanntmachung des Hinweises auf die Patenterteilung: **09.03.83 Patentblatt 83/10**

(84) Benannte Vertragsstaaten: **BE DE FR GB IT**

(56) Entgegenhaltungen:
**EP A 0 005 617**
**CH A 550 130**
**DE A 2 559 255**
**US A 3 912 672**

**Chemical Abstracts Band 69, Nr. 8, 19. August 1968 Columbus, Ohio, USA A. KABI et al. « Gamma-Radiolysis of aqueous solutions of arylkylamines » Seite 2986, Spalte 1, Abstract Nr. 32003u.**

(73) Patentinhaber: **BAYER AG**
**Zentralbereich Patente, Marken und Lizenzen**
**D-5090 Leverkusen 1, Bayerwerk (DE)**

(72) Erfinder: **Mayer, Wolfram, Dr.**
**Mozartstrasse 10**
**D-4150 Krefeld 1 (DE)**
Erfinder: **de Cleur, Eckhard, Dr.**
**Aubruchsgraben 14**
**D-4100 Duisburg 46 (DE)**
Erfinder: **Rudolph, Hans, Dr.**
**Haydnstrasse 9**
**D-4150 Krefeld 1 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Ammoniumsalze, ein Verfahren zur Herstellung der ihnen zugrundeliegenden Amine, sowie die Ammoniumsalze enthaltende Beschichtungsmassen auf Basis von Polyurethan- oder Epoxiharzvorprodukten

Die vorliegende Erfindung betrifft neue Ammoniumsalze von Carbonsäuren, ein Verfahren zur Herstellung der ihnen zugrundeliegenden Amine, sowie diese Ammoniumsalze als latente Katalysatoren enthaltende Beschichtungsmassen.

Tertiäre Amine stellen bekanntlich in der Praxis vielfach verwendeter Beschleuniger für zu Polyurethanen bzw. Polyepoxiden ausreagierende Reaktionsgemische dar. Für viele Anwendungsgebiete ist es erwünscht, nicht die freien Amine einzusetzen, sondern die Amine dem Reaktionsgemisch in einer verkappten Form zuzusetzen, aus welcher sie zu einem definierten Zeitpunkt freigesetzt werden, wodurch die gewünschte Reaktion gestartet wird.

So ist es z. B. möglich aus quartären Ammoniumsalzen durch Erhitzen auf über 100 °C Amine in Freiheit zu setzen (Houben-Weyl, Methoden der organischen Chemie, Bd. XI,1). In vielen Fällen ist es jedoch erwünscht, die Amine schon bei wesentlich niedrigeren Temperaturen zu deblockieren.

Überraschenderweise wurde nunmehr gefunden, daß aus Ammoniumsalzen bestimmter Carbonsäuren die Amine auch bei Raumtemperatur unter Einwirkung von Licht der Wellenlänge 250-500 nm unter Decarboxylierung der Carbonsäure freigesetzt werden. Diese, nachstehend näher beschriebenen, erfindungsgemäßen Ammoniumsalze stellen somit wertvolle latente Beschleuniger für Reaktivsysteme auf Basis von Polyurethan- oder Polyepoxid-Vorprodukten dar.

Gegenstand der vorliegenden Erfindung sind Ammoniumsalze, ausgewählt aus der Gruppe bestehend aus

a) Verbindungen der Formel

$$A—(0)_m—(CH_2)_n—COO^{(-)}\quad Kat^{(+)}$$

in welcher

m und n gleich oder verschieden sind und für 0 oder 1 stehen, mit der Einschränkung, daß im Falle n = 0, m ebenfalls für 0 steht,

A für einen gegebenenfalls Cyano-, Nitro-, Fluor-, Chlor-, Trifluormethyl-, ($C_1$-$C_4$-Alkoxy)-carbonyl- oder ($C_1$-$C_4$-alkyl)-carbonyl-substituierten Phenyl-, 1-Naphthyl- oder Anthracyl-Rest steht oder einen gegebenenfalls Methyl-, Phenyl- oder Cyano-Substituenten und Stickstoff als Heteroatom aufweisenden heterocyclischen Rest mit 4-10 Kohlenstoffatomen steht, mit der Einschränkung, daß im Falle von m + n = 0 A einen, gegebenenfalls einen der genannten Substituenten aufweisenden Anthracyl-Rest oder einen gegebenenfalls einen der genannten Substituenten aufweisenden heterocyclischen Rest der genannten Art bedeutet und

$Kat^{(+)}$ für ein Kation steht, wie es durch Anlagerung eines Protons an ein gegebenenfalls Hydroxyl-, Mercapto-, Ether-, Thioether-, Amid-, Ester- oder Halogen-Gruppen aufweisendes, die Isocyanat-Polyadditionsreaktion beschleunigendes tertiäres Amin des Molekulargewichtsbereichs 59-200 erhalten wird,

b) Salzen der m-Methoxyphenylessigsäure und der oben bei der Definition von $Kat^{(+)}$ genannten Amine und

c) dem Dimethylbenzylamin-Salz der p-Methoxyphenylessigsäure.

Gegenstand der vorliegenden Erfindung ist auch ein Verfahren zur Herstellung von tertiären Aminen, welches dadurch gekennzeichnet ist, daß man diese Ammoniumsalze mit Licht einer Wellenlänge zwischen 250 und 500 nm bestrahlt.

Gegenstand der vorliegenden Erfindung sind schließlich auch Beschichtungsmassen auf Basis von in Gegenwart von Aminen aushärtenden Polyurethan- oder Epoxiharz-Vorprodukten, dadurch gekennzeichnet, daß sie 0,1 bis 10 Gew.-%, bezogen auf Feststoffgehalt, eines der genannten Ammoniumsalze enthalten.

Die CH-A-550 130 beschreibt zwar Salze von primären, sekundären oder tertiären Aminen der 2-Methyl-4-chlorphenoxyessigsäure, welche als Wirkstoff für herbizide Mittel geeignet sind, sie vermittelt jedoch keinerlei Hinweis, daß die neuen erfindungsgemäßen Ammoniumsalze auf Basis von tertiären Aminen und keine Alkylsubstituenten aufweisenden aromatischen bzw. araliphatischen Säuren wertvolle latente Katalysatoren für Beschichtungsmassen auf Basis von Polyurethan- oder Epoxyharzvorprodukten darstellen.

Auch die US-A-3 912 672, die quaternäre Ammoniumsalze anorganischer oder organischer Säuren als Vulkanisationsbeschleuniger für Acrylatkautschuk empfiehlt, vermittelt keinerlei Hinweis in diese Richtung. Die gleiche Aussage rechtfertigt sich im Hinblick auf die Lehre der US-A-3 980 699, die u. a. Salze tertiärer Amine der 2-Naphthylessigsäure als pharmazeutisch wirksame Substanzen beschreibt.

Besonders bevorzugte Ammoniumsalze sind solche der oben unter a) genannten allgemeinen Formel, für welche

A einen gegebenenfalls Cyano-, Nitro-, Fluor-, Chlor-, Trifluormethyl-, ($C_1$-$C_4$-Alkoxy)-carbonyl- oder ($C_1$-$C_4$-Alkyl)-carbonyl-substituierten Phenyl-, 1-Naphthyl- oder Anthracylrest bedeutet und

m für 0 und n für 1 steht und

$Kat^{(+)}$ die bereits obengenannte Bedeutung hat.

Die erfindungsgemäßen Ammoniumsalze stellen somit Neutralisationsprodukte von Säuren dar wie z. B.

Phenylessigsäure, o-, m-, p-Chlorphenylessigsäure, p-Cyanphenylessigsäure, m-, p-Nitrophenylessigsäure, m-Methoxyphenylessigsäure, Dinitrophenylessigsäure, 1-Naphthylessigsäure, Anthracen-9-carbonsäure, Phenoxyessigsäure, o-, m-, p-Cyanophenoxyessigsäure, 2,4-Dichlorphenoxyessigsäure, 2, 4-Dicyanphenoxyessigsäure, m-, p-Nitrophenoxyessigsäure, 2,4-Dinitrophenoxyessigsäure, 2-Pyridylessigsäure, 2-Phenyl-6-methyl-chinolin-4-carbonsäure oder 2-Methyl-3-cyan-chinolin-4-carbonsäure.

Besonders geeignet sind solche Säuren, deren pKa-Wert kleiner als 4 ist, bevorzugt zwischen 1 und 3 liegt.

Erfindungsgemäß bevorzugt sind :

Phenylessigsäure, p-Nitrophenylessigsäure, 1-Naphthylessigsäure, Anthracen-9-carbonsäure, p-Cyanphenoxyessigsäure, 2-Phenyl-6-methylchinolin-4-carbonsäure.

Das in den erfindungsgemäßen Ammoniumsalzen vorliegende Kation entsteht durch Anlagerung eines Protons an ein tertiäres Amin. Bei diesem tertiären Amin handelt es sich um ein, die Isocyanats-Polyadditionsreaktion beschleunigendes tertiäres Amin des Molekulargewichtsbereichs 59-200. Derartige, die Isocyanat-Polyadditionsreaktion beschleunige tert. Amine weisen im allgemeinen auch eine beschleunigende Wirkung auf die Aushärtung von Epoxiharz-Vorprodukten auf. Grundsätzlich können zur Herstellung der erfindungsgemäßen Ammoniumsalze beliebige tertiäre Amine eingesetzt werden, die im allgemeinen 1 bis 5, vorzugsweise 1 bis 3 und insbesondere 1 bis 2 tert. Aminoatome aufweisen. Grundsätzlich ist es denkbar, daß die Amine auch noch andere funktionelle Gruppen wie z. B. Hydroxyl-, Mercapto-, Ether-, Thioether-, Amid- und Estergruppen oder auch Halogenatome aufweisen.

Beispiele für erfindungsgemäß in Frage kommende Amine sind tertiäre Amine, wie Triethylamin, Tributylamin, N-Methyl-morpholin, N-Ethyl-morpholin, N,N,N', N'-Tetramethyl-ethylendiamin, Pentamethyl-diethylentriamin und höhere Homologe (DE-Offenlegungsschriften 2 624 527 und 2 624 528), 1,4-Diazabicyclo-(2,2,2)-octan, N-Methyl-N'-dimethylaminoethylbicyclo-(2,2,2)-octan, N-Methyl-N'-dimethylaminoäthylpiperazin, Bis-(dimethylaminoalkyl)-piperazine (DE-Offenlegungsschrift 2 636 787), N,N-Dimethylbenzylamin, N,N-Dimethylcyclohexylamin, N,N-Diäthylbenzylamin, Bis-(N,N-diäthylaminoäthyl)-adipat, N,N,N', N'-Tetramethyl-1,3-butandiamin, N,N-Dimethyl-β-phenyläthylamin, 1,2-Dimethylimidazol, 2-Methylimidazol, monocyclische und bicyclische Amidine (DE-Offenlegungsschrift 1 720 633), Bis-(dialkylamino)alkyl-äther (US-Patentschrift 3 330 782, DE-Auslegeschrift 1 030 558, DE-Offenlegungsschriften 1 804 361 und 2 618 280) sowie Amidgruppen (vorzugsweise Formamidgruppen) aufweisende tertiäre Amine gemäß den DE-Offenlegungsschriften 2 523 633 und 2 732 929) ; aktive Wasserstoffatome aufweisende tertiäre Amine, z. B. Triäthanolamin, Triisopropanolamin, N-Methyl-diäthanolamin, N-Äthyldiäthanolamin, N,N-Dimethyl-äthanolamin, deren Umsetzungsprodukte mit Alkylenoxiden wie Propylenoxid und/oder Äthylenoxid sowie sekundär-tertiäre Amine gemäß DE-Offenlegungsschrift 2 732 292 ; ferner Silaamine mit Kohlenstoff-Silizim-Bindungen, wie sie z. B. in der DE-Patentschrift 1 229 290 (entsprechend der US-Patentschrift 3 620 984) beschrieben sind, z. B. 2,2,4-Trimethyl-2-silamorpholin und 1,3-Diäthylaminomethyl-tetramethyldisiloxan.

Erfindungsgemäß bevorzugte Amine sind tert.-Butylamin, Triethylamin, Tributylamin, N,N-Dimethylbenzylamin, Diazabicyclooctan (DABCO) und Diazabicycloundecen (DBU).

Die erfindungsgemäßen Ammoniumsalze sind im allgemeinen kristalline Verbindungen mit definiertem Schmelzpunkt. Sie sind in vielen organischen Lösungsmitteln löslich, z. B. in Aceton, Acetonitril, Chloroform, Methylenchlorid, Methanol, Äthanol und Äthylenglykolmonomethylätheracetat.

Die Erfindungsgemäßen Ammoniumsalze können in einfacher Weise hergestellt werden, indem man die Carbonsäuren der genannten Formel bei Temperaturen von − 20 bis + 50 °C, vorzugsweise bei Raumtemperatur, gegebenenfalls in Anwesenheit eines organischen Lösungsmittels wie Ether, Benzol, Toluol, Chlorbenzol, Petrolether, Aceton, Dioxan, Chloroform etc., mit der Aminkomponente umsetzt. Im allgemeinen wird dabei pro Aminäquivalent etwa 1 Äquivalent der Carbonsäure eingesetzt. Bei vielen Diaminen (z. B. Diazabicyclooctan) sind auch die Salze mit nur 1 Äquivalent an Carbonsäure (also die Monoammoniumsalze) als erfindungsgemäße latente Initiatoren brauchbar. Auch solche nur teilweise mit Carbonsäuren der genannten Formel neutralisierte Polyamine werden daher von der vorliegenden Erfindung mitumfaßt. Im übrigen läßt sich durch einen einfachen Vorversuch feststellen, wieviele Aminstickstoffatome eines Polyamins in die Ammoniumsalzform übergeführt werden müssen, um die gewünschte Desaktivierung zu erreichen.

Wie schon erwähnt, können aus den erfindungsgemäßen Ammoniumsalzen unter photochemischer Spaltung die zugrundeliegenden Amine zu jedem gewünschten Zeitpunkt unter sehr milden Bedingungen (schon bei Raumtemperatur) durch Bestrahlen mit Licht einer Wellenlänge zwischen 250 und 500 nm, vorzugsweise 300-400 nm in Freiheit gesetzt werden.

Es ist erfindungsgemäß vorteilhaft, die Photoreaktion durch Zusatz von an sich bekannten Triplett-Sensibilisatoren wie z. B. Benzophenon, Acetophenon, Propiophenon, Xanthon, Thioxanthon oder Triphenylen zu sensibilisieren. Diese Sensibilisatoren werden vorzugsweise in einer Menge von 0,1 bis 30 Gew.-%, besonders bevorzugt 3 bis 10 Gew.-%, bezogen auf Ammoniumsalz, zugegeben.

Die erfindungsgemäßen Ammoniumsalze sind insbesondere als durch UV-Strahlen aktivierbare Härtungskatalysatoren für Harze oder Harzkompositionen geeignet. Solche Harze sind z. B. Epoxyharze oder Lacke auf Basis von Isocyanaten, die entweder als solche, in chemisch modifizierter Form oder in

Mischung mit Harzen anderer Art verwendet werden.

Nach dem Isocyanat-Polyadditions-Verfahren aus Polyhydroxylverbindungen und Polyisocyanaten erhaltene Lacküberzüge mit vernetzter hochmolekularer Polyurethanstruktur sind bekannt. Diese technisch wertvollen Überzüge lassen sich sowohl von Hand als auch maschinell, z. B. durch Spritzen, Tauchen oder Gießen, auftragen. In der Praxis wird entweder das sogenannte Zweikomponenten- oder das Einkomponenten-Verfahren angewandt. Im ersteren Fall werden die beiden Komponenten (Polyisocyanat einerseits ; gegenüber Isocyanaten reaktive Verbindung andererseits), gegebenenfalls in Anwesenheit eines Lösungsmittels, vermischt. Eine Reaktion der beiden Komponenten läßt sich dabei nur dann vollständig vermeiden, wenn man verkappte Polyisocyanate einsetzt, die erst beim Erhitzen das freie Isocyanat abspalten ; die Anwendung solcher Systeme ist aber auf einzubrennende Lackierungen beschränkt. Immerhin haben auch die Lackmischungen, welche freie Polyisocyanate enthalten, eine mehr oder weniger lange Lebensdauer (Pot-life), die es ermöglicht, diese Lacke in technisch befriedigender Weise von Hand aus oder maschinell auf die Unterlagen aufzutragen, auf denen sie dann unter Polyurethanbildung endgültig aushärter und vernetzen. Einkomponentensysteme enthalten dagegen ein Addukt mit freien Isocyanatgruppen aus Polyhydroxylverbindung und einem Überschuß an Polyisocyanat, wobei nach dem Auftrag durch Reaktion der freien NCO-Gruppen im Lack mit Wasser (Luftfeuchtigkeit) Vernetzung erfolgt. Auch hierbei muß man dafür Sorge tragen, daß eine vorzeitige Vernetzung während der Lagerung des Lackes unterbleibt, indem man die Luftfeuchtigkeit ausschließt und/oder wasserbeseitigende Mittel zusetzt.

Andererseits ist es erwünscht, daß nach dem Auftragen des Lackes auf die Unterlage eine möglichst schnelle Vernetzung und Trocknung erfolgt. Sowohl bei Einkomponentenals auch bei Zweikomponentenlacken ist es möglich, durch den Zusatz von an sich bekannten Reaktionsbeschleunigern die Härtungsreaktion zu fördern. Die Forderung der beschleunigten Vernetzungsreaktion auf der Unterlage widerspricht aber der gleichzeitig zu erhebenden Forderung nach einer möglichst langen Verarbeitungsfähigkeit. Im Prinzip wäre es denkbar, der Lackmischung einen Katalysator erst kurz vor der Applikation auf die Unterlage zuzufügen. Das mag zwar bei einem Handverfahren im kleinen Maßstab möglich sein, verbietet sich aber bei großtechnischer, maschineller Arbeitsweise, da die Lackmischungen in der Maschine längere Verweilzeiten, zum Teil bei höherer Temperatur, besitzen und eine ungewollte Verkürzung der Verarbeitungsspanne (Pot-life) durch die beschleunigende Wirkung des Katalysators nicht zu vermeiden ist. Man hat auch bereits daran gedacht, den Katalysator noch nachträglich nach dem Auftragen der Lackmischung auf die Unterlage, etwa durch Aufdüsen im gasförmigen Zustand, aufzubringen ; das aber erfordert aufwendige zusätzliche maschinelle Einrichtungen. Überdies sind zwangsläufig nur einige wenige Katalysatoren auf diese Weise verarbeitbar.

Aus der DE-OS 1 621 883 ist es bekannt, auf die zu lackierende Oberfläche zunächst eine Lackschicht aus einem physikalisch trocknenden, einen für Isocyanat-Polyadditions-Reaktionen an sich bekannten Katalysator enthaltenden Bindemittel und anschließend einen katalysatorfreien Polyurethanlack aufzubringen. Auf diese Weise gelingt es, ohne Beeinträchtigung der Lagerstabilität und der Verarbeitungsfähigkeit einer Polyurethanlackmischung die Trocknungszeit des Lacks erheblich herabzusetzen. Nachteilig ist dabei aber der zusätzliche Aufwand der zweiten Lackierung.

Prinzipiell dieselben Probleme wie bei den oben geschilderten Polyurethan-Einkomponenten- und Zweikomponentensystemen treten auch bei Beschichtungsmassen auf Basis von Epoxyharzen auf, deren Vernetzung im allgemeinen ebenfalls mittels tertiärer Amine katalysiert werden muß. In der DE-OS 2 357 859 wird vorgeschlagen, zu diesem Zweck den Beschichtungsmassen Salze von tertiären Aminen mit bestimmten $\alpha$-substituierten Carbonsäuren zuzusetzen. Beim Erhitzen auf 70 bis 200 °C zersetzen sich diese Ammoniumsalze unter Decarboxylierung, wonach unter dem katalytischen Einfluß des freiwerdenden Amins die Beschichtung rasch aushärtet.

Mit den erfindungsgemäßen Ammoniumsalzen werden nunmehr verkappte tertiäre Aminkatalysatoren zur Verfügung gestellt, welche eine rasche Aushärtung der Beschichtung schon bei Raumtemperatur durch Bestrahlen mit kurzwelligem Licht ermöglichen und welche Lackierungen mit besonders glänzender Oberfläche ergeben. Andererseits können aus den erfindungsgemäßen Ammoniumsalzen die Amine auch durch Erwärmen auf ca. 50-130 °C in Freiheit gesetzt werden.

In den erfindungsgemäßen Beschichtungsmassen liegen die als latente Beschleuniger wirkenden erfindungsgemäßen Ammoniumsalze in einer Menge von 0,1 bis 10 Gew.-%, vorzugsweise 0,3 bis 8 Gew.-% und insbesondere 0,9 bis 6 Gew.-%, bezogen auf Feststoff, vor.

Als Grundlage für die erfindungsgemäßen Beschichtungsmassen kommen die in Gegenwart von Aminen vernetzenden Einkomponenten- und Zweikomponentenpolyurethansysteme in Betracht, wie sie in der Lack- und Beschichtungstechnik an sich bekannt sind. Einkomponentensysteme sind, wie bereits oben kurz erwähnt, gegebenenfalls in inerten organischen Lösungsmitteln gelöste Vorpolymerisate mit einem Gehalt von ca. 1 bis 25 Gew.-%, vorzugsweise 2,5 bis 19 Gew.-%, an freien NCO-Gruppen, welche durch Umsetzung von höhermolekularen und/oder niedermolekularen Verbindungen mit gegenüber Isocyanaten reaktiven Gruppen, wie sie nachstehend beschrieben werden, mit einem Überschuß an den nachstehend beschriebenen Polyisocyanaten hergestellt wurden. Bei Zweikomponentenpolyurethanen handelt es sich um ein gegebenenfalls in einem inerten organischen Lösungsmittel gelöstes Gemisch aus einer höhermolekularen Polyhydroxylverbindung, (z. B. einem Hydroxylgruppen aufweisenden Vorpolymeren aus Polyisocyanaten und einem Überschuß an Polyolen) einerseits und einem Polyisocyanat

andererseits.

Für die erfindungsgemäßen Beschichtungsmassen kommen aliphatische, cycloaliphatische, araliphatische, aromatische und heterocyclische Polyisocyanate in Frage, wie sie z. B. von W. Siefken in Justus Liebigs Annalen der Chemie, 562, Seiten 75 bis 136, beschrieben werden, beispielsweise solche der Formel

$$Q\,(NCO)_n$$

in der

n = 2-4, vorzugsweise 2, und

Q einen aliphatischen Kohlenwasserstoffrest mit 2-18, vorzugsweise 6-10 C-Atomen, einen cycloaliphatischen Kohlenwasserstoffrest mit 4-15, vorzugsweise 5-10 C-Atomen, einen aromatischen Kohlenwasserstoffrest mit 6-15, vorzugsweise 6-13 C-Atomen, oder einen araliphatischen Kohlenwasserstoffrest mit 8-15, vorzugsweise 8-13 C-Atomen, bedeuten, z. B. Äthylen-diisocyanat, 1,4-Tetramethylendiisocyanat, 1,6-Hexamethylendiisocyanat, Trimethylhexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat sowie Naphthylen-1,5-diisocyanat.

Ferner kommen beispielsweise erfindungsgemäß in Frage : Triphenylmethan-4,4',4''-triisocyanat, Polyphenyl-polymethylen-polyisocyanate, wie sie durch Anilin-Formaldehyd-Kondensation und anschließende Phosgenierung erhalten und z. B. in den GB-Patentschriften 874 430 und 848 671 beschrieben werden, m- und p-Isocyanatophenylsulfonyl-isocyanate gemäß der US-Patentschrift 3 454 606, perchlorierte Arylpolyisocyanate, wie sie z. B. in der DE-Auslegeschrift 1 157 601 (US-Patentschrift 3 277 138) beschrieben werden, Carbodiimidgruppen aufweisende Polyisocyanate, wie sie in der DE-Patentschrift 1 092 007 (US-Patentschrift 3 152 162) sowie in den DE-Offenlegungsschriften 2 504 400, 2 537 685 und 2 552 350 beschrieben werden, Norbornan-Diisocyanate gemäß US-Patentschrift 3 492 330, Allophanatgruppen aufweisende Polyisocyanate, wie sie z. B. in der GB-Patentschrift 994 890, der BE-Patentschrift 761 626 und der NL-Patentanmeldung 7 102 524 beschrieben werden, Isocyanuratgruppen aufweisende Polyisocyanate, wie sie z. B. in der US-Patentschrift 3 001 973, in den DE-Patentschriften 1 022 789, 1 222 067 und 1 027 394 sowie in den DE-Offenlegungsschriften 1 929 034 und 2 004 048 beschrieben werden, Urethangruppen aufweisende Polyisocyanate, wie sie z. B. in der BE-Patentschrift 752 261 oder in den US-Patentschriften 3 394 164 und 3 644 457 beschrieben werden, acylierte Harnstoffgruppen aufweisende Polyisocyanate gemäß der DE-Patentschrift 1 230 778, Biuretgruppen aufweisende Polyisocyanate, wie sie z. B. in den US-Patentschriften 3 124 605, 3 201 372 und 3 124 605 sowie in der GB-Patentschrift 889 050 beschrieben werden, durch Telomerisationsreaktionen hergestellte Polyisocyanate, wie sie z. B. in der US-Patentschrift 3 654 106 beschrieben werden, Estergruppen aufweisende Polyisocyanate, wie sie z. B. in den GB-Patentschriften 965 474 und 1 072 956, in der US-Patentschrift 3 567 763 und in der DE-Patentschrift 1 231 688 genannt werden, Umsetzungsprodukte der obengenannten Isocyanate mit Acetalen gemäß der DE-Patentschrift 1 072 385 und polymere Fettsäureester enthaltende Polyisocyanate gemäß der US-Patentschrift 3 455 883.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Bevorzugte Polyisocyanate sind Hexamethylendiisocyanat, dessen Isocyanurat und dessen Biuret ; 1-Isocyanato-3,3,5-trimethyl-5-isocyanatomethyl-cyclohexan (Isophorondiisocyanat) ; die Toluylendiisocyanate und deren Isocyanurate ; das Mischisocyanurat aus Toluylendiisocyanat und Hexamethylendiisocyanat ; das Umsetzungsprodukt aus 1 Mol Trimethylolpropan und 3 Mol Toluylendiisocyanat sowie rohes Diphenylmethandiisocyanat.

Geeignete höhermolekulare Verbindungen mit mindestens zwei gegenüber Isocyanaten reaktionsfähigen Wasserstoffatomen sind solche mit einem Molekulargewicht in der Regel von 400 bis 50 000. Hierunter versteht man neben Aminogruppen, Thiolgruppen oder Carboxylgruppen aufweisenden Verbindungen vorzugsweise Hydroxylgruppen aufweisende Verbindungen, insbesondere zwei bis acht Hydroxylgruppen aufweisende Verbindungen, speziell solche vom Molekulargewicht 500 bis 25 000, vorzugsweise 700 bis 20 000, z. B. mindestens zwei, in der Regel 2 bis 8, vorzugsweise aber 2 bis 4, Hydroxylgruppen aufweisende Polyester, Polyether, Polythioether, Polyacetale, Polycarbonate, Polyacrylate, Polyesteramide und OH-Präpolymere, wie sie für die Herstellung von honogenen und von zellförmigen Polyurethanen an sich bekannt sind :

a) Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

Als Beispiele für solche Carbonsäuren und deren Derivate seien genannt :

Bernsteinsäure, Adipinsäure, Korksäure, Azelainsäure, Sebacinsäure, Phthalsäure, Isophthalsäure, Terephthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimerisierte und trimerisierte ungesättigte Fettsäuren, gegebenenfalls in Mischung mit monomeren ungesättigten Fettsäuren, wie Ölsäure ; Terephthalsäuredimethylester und Terephthalsäure-bis-glykolester. Als mehrwertige Alkohole kommen z. B. Äthylenglykol, Propylenglykol-(1,2) und -(1,3), Butylenglykol-(1,4) und -(2,3), Hexandiol-(1,6), Octandiol-(1,8), Neopentylglykol, 1,4-Bis-hydroxymethylcyclohexan, 2-Methyl-1,3-propandiol, Glycerin, Trimethylolpropan, Hexantriol-(1,2,6), Butantriol-(1,2,4), Trimethyloläthan, Pentaerythrit, Chinit, Mannit und Sorbit, Formit, Methylglykosid, ferner Diäthylenglykol, Triäthylenglykol, Tetraäthylenglykol und höhere Polyäthylenglykole, Dipropylenglykol und höhere Polypropylenglykole sowie Dibutylenglykol und höhere Polybutylenglykole in Frage. Die Polyester können anteilig endständige Carboxylgruppen aufweisen. Auch Polyester aus Lactonen, z. B. ε-Caprolacton, oder aus Hydroxycarbonsäuren, z. B. ω-Hydroxycapronsäure, sind einsetzbar.

b) Auch die erfindungsgemäß in Frage kommenden, mindestens zwei, in der Regel zwei bis acht, vorzugsweise zwei bis drei, Hydroxylgruppen aufweisenden Polyäther sind solche der an sich bekannten Art und werden z. B. durch Polymerisation von Epoxiden wie Äthylenoxid, Propylenoxid, Butylenoxid, Tetrahydrofuran, Styroloxid oder Epichlorhydrin mit sich selbst, z. B. in Gegenwart von Lewis-Katalysatoren wie $BF_3$, oder durch Anlagerung dieser Epoxide, vorzugsweise von Äthylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z. B. Äthylenglykol, Propylenglykol-(1, 3) oder -(1,2), Trimethylolpropan, Glycerin, Sorbit, 4,4'-Dihydroxy-diphenylpropan, Anilin, Äthanolamin oder Äthylendiamin hergestellt. Auch Sucrosepolyäther, wie sie z. B. in den DE-Auslegeschriften 1 176 358 und 1 064 938 beschrieben werden, sowie auf Formit oder Formose gestartete Polyäther (DE-Offenlegungsschriften 2 639 083 bzw. 2 737 951), kommen erfindungsgemäß in Frage. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch OH-Gruppen aufweisende Polybutadiene sind erfindungsgemäß geeignet.

c) Unter den Polythioäthern seien insbesondere die kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten z. B. um Polythiomischäther, Polythioätherester oder Polythioätheresteramide.

d) Als Polyacetale kommen z. B. die aus Glykolen, wie Diäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale wie z. B. Trioxan (DE-Offenlegungsschrift 1 694 128) lassen sich erfindungsgemäß geeignete Polyacetale herstellen.

e) Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diäthylenglykol, Triäthylenglykol, Tetraäthylenglykol oder Thiodiglykol mit Diarylcarbonaten, z. B. Diphenylcarbonat, oder Phosgen hergestellt werden können (DE-Auslegeschriften 1 694 080, 1 915 908 und 2 221 751 ; DE-Offenlegungsschrift 2 605 024).

f) Zu den Polyesteramiden und Polyamiden zählen z. B. die aus mehrwertigen gesättigten oder ungesättigten Carbonsäuren bzw. deren Anhydriden und mehrwertigen gesättigten oder ungesättigten Aminoalkoholen, Diaminen, Polyaminen und deren Mischungen gewonnenen, vorwiegend linearen Kondensate.

g) Acrylatharze, welche als hydroxylgruppenhaltige Komponente verwendet werden können, sind Homo- oder Copolymerisate mit mindestens zwei Hydroxylgruppen pro Molekül, wobei z. B. folgende Monomere als Ausgangsprodukte gewählt werden können :

Ester der Acrylsäure und Methacrylsäure mit zweiwertigen, gesättigten, aliphatischen Alkoholen mit 2-4 C-Atomen, wie z. B. 2-Hydroxyäthylacrylat, 2-Hydroxypropylacrylat, 4-Hydroxybutylacrylat und die entsprechenden Methacrylsäureester ; Acrylsäure und Methacrylsäure ; Acrylsäure- und Methacrylsäurealkylester mit 1-18, vorzugsweise 1-8 C-Atomen in der Alkoholkomponente, wie z. B. Methylacrylat, Äthylacrylat, Propylacrylat, Isopropylacrylat, n-Butylacrylat, tert.-Butylacrylat, 2-Äthylhexylacrylat, Stearylacrylat und die entsprechenden Methacrylsäureester ; Acrylsäure- und Methacrylsäurecyclohexylester ; Acrylnitril und Methacrylnitril ; Acrylamid und Methacrylamid ; N-Methoxymethyl(meth)acrylsäureamid.

h) Auch bereits Urethan-, polymerisationsfähige CC-Doppelbindungen und/oder Harnstoffgruppen enthaltende Polyhydroxylverbindungen sowie gegebenenfalls modifizierte natürliche Polyole, wie Rizinusöl oder Kohlenhydrate, z. B. Stärke, sind verwendbar. Auch Anlagerungsprodukte von Alkylenoxiden an Phenol-Formaldehyd-Harze oder auch an Harnstoff-Formaldehydharze sind erfindungsgemäß einsetzbar.

i) Die genannten Polyhydroxylverbindungen können vor ihrer Verwendung im Polyisocyanat-Polyadditionsverfahren noch in der verschiedensten Weise modifiziert werden: So läßt sich gemäß DE-Offenlegungsschriften 2 210 839 (US-Patentschrift 3 849 515) und 2 544 195 ein Gemisch aus verschiedenen Polyhydroxylverbindungen (z. B. aus einem Polyäther- und einem Polyesterpolyol) durch Verätherung in Gegenwart einer starken Säure zu einem höhermolekularen Polyol kondensieren, welches aus über Ätherbrücken verbundenen verschiedenen Segmenten aufgebaut ist.

j) Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) in situ in den oben genannten, Hydroxylgruppen aufweisenden Verbindungen ablaufen läßt. Derartige Verfahren sind beispielsweise in den DE-Auslegeschriften 1 168 075 und 1 260 142, sowie den DE-Offenlegungsschriften 2 324 134, 2 423 984, 2 512 385, 2 513 815, 2 550 796, 2 550 797, 2 550 833, 2 550 862, 2 633 293 und 2 639 254 beschrieben. Es ist aber auch möglich, gemäß US-Patentschrift 3 869 413 bzw. DE-Offenlegungsschrift 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z. B. durch Polymerisation von Styrol und Acrylnitril in Gegenwart von Polyäthern (US-Patentschriften 3 383 351, 3 304 273, 3 523 093, 3 110 695; DE-Auslegeschrift 1 152 536) oder Polycarbonatpolyolen (DE-Patentschrift 1 769 795; US-Patentschrift 3 637 909) erhalten werden, sind erfindungsgemäß geeignet. Bei Verwendung von Polyätherpolyolen, welche gemäß den DE-Offenlegungsschriften 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit. Polyhydroxylverbindungen, in welche durch radikalische Pfropfpolymerisation mittels ungesättigter Carbonsäuren sowie gegebenenfalls weiterer olefinisch ungesättigter Monomerer Carboxylgruppen eingeführt wurden (DE-Offenlegungsschriften 2 714 291, 2 739 620 und 2 654 746) können mit besonderem Vorteil in Kombination mit mineralischen Füllstoffen eingesetzt werden.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Vertreter der genannten erfindungsgemäß zu verwendenden Verbindungen sind z. B. in High Polymers, Vol. XVI, « Polyurethanes, Chemistry and Technology », verfaßt von Saunders-Frisch, Interscience Publishers, New York, London, Band I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München, 1966, z. B. auf den Seiten 45-71, beschrieben. Selbstverständlich können Mischungen der obengenannten Verbindungen mit mindestens zwei gegenüber Isocyanaten reaktionsfähigen Wasserstoffatomen mit einem Molekulargewicht von 400-50 000, z. B. Mischungen von Polyäthern und Polyestern, eingesetzt werden.

Von besonderem Vorteil ist es dabei in manchen Fällen, niedrigschmelzende und hochschmelzende Polyhydroxylverbindungen miteinander zu kombinieren (DE-Offenlegungsschrift 2 706 297).

Erfindungsgemäß geeignete niedermolekulare Verbindungen mit mindestens zwei gegenüber Isocyanaten reaktionsfähigen Wasserstoffatomen (Molekulargewicht von 32 bis 400) sind ebenfalls vorzugsweise Hydroxylgruppen aufweisende Verbindungen mit in der Regel 2 bis 8, vorzugsweise 2 bis 4, gegenüber Isocyanaten reaktionsfähigen Wasserstoffatomen.

Auch in diesem Fall können Mischungen von verschiedenen Verbindungen mit mindestens zwei gegenüber Isocyanaten reaktionsfähigen Wasserstoffatomen mit einem Molekulargewicht von 32 bis 400 verwendet werden.

Als Beispiele für derartige Verbindungen seien genannt:

Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethylcyclohexan, 2-Methyl-1,3-propandiol, Dibrombutendiol (US-Patentschrift 3 723 392), Glyzerin, Trimethylolpropan, Hexantriol-(1,2,6), Trimethyloläthan, Pentaerythrit, Chinit, Mannit und Sorbit, Ricinusöl, Diäthylenglykol, Triäthylenglykol, Tetraäthylenglykol, höhere Polyäthylenglykole mit einem Molekulargewicht bis 400, Dipropylenglykol, höhere Polypropylenglykole mit einem Molekulargewicht bis 400, Dibutylenglykol, höhere Polybutylenglykole mit einem Molekulargewicht bis 400, 4,4′-Dihydroxy-diphenylpropan und Di-hydroxymethylhydrochinon.

Als niedermolekulare Polyole kommen erfindungsgemäß auch die Gemische von Hydroxyaldehyden und Hydroxyketonen (« Formose ») bzw. die hieraus durch Reduktion erhaltenen mehrwertigen Alkohole (« Formit ») in Frage, wie sie bei der Selbstkondensation von Formaldehydhydrat in Gegenwart von Metallverbindungen als Katalysator und von zur Endiolbildung befähigten Verbindungen als Co-Katalysator entstehen (DE-Offenlegungsschriften 2 639 084, 2 714 084, 2 714 104, 2 721 186, 2 738 154 und 2 738 512). Auch Lösungen von Polyisocyanatpolyadditionsprodukten, insbesondere von ionische Gruppen aufweisenden Polyurethanharnstoffen und/oder von Polyhydrazodicarbonamiden, in niedermolekularen, mehrwertigen Alkoholen kommen erfindungsgemäß als Polyolkomponente in Betracht (DE-Offenlegungsschrift 2 638 759).

Erfindungsgemäß geeignete Beschichtungssysteme auf Basis von Epoxiharz-Vorprodukten sind beispielsweise Triglycidylurazol, Triglycidylisocyanurat ; Polyepoxide mit Molgewichten bis zu 2 000, wie sie aus Bisphenol A und Epichlorhydrin erhalten werden ; Bisglycidylester der Terephthalsäure, der Isophthalsäure, der Phthalsäure, Tetrahydrophthalsäure, Hexahydrophthalsäure oder Hexahydroterephthalsäure ; Trisglycidylester der Trimellitsäure ; Tetraglycidylester und β-Methylglycidylester der Pyromellitsäure, Glycidylderivate des Hydantoins gemäß der Formel

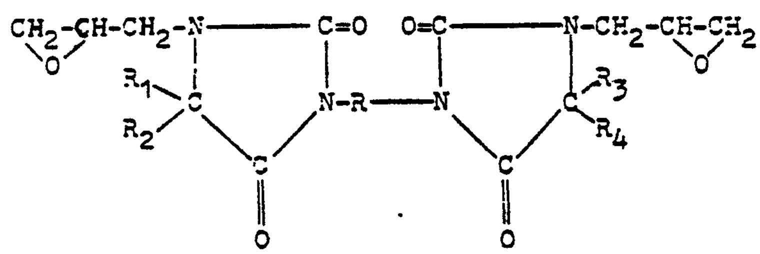

worin

R einen zweiwertigen aliphatischen, cycloaliphatischen oder araliphatischen Rest darstellt und

$R_1$, $R_2$, $R_3$ und $R_4$ je ein Wasserstoffatom oder einen aliphatischen oder cycloaliphatischen Kohlenwasserstoffrest bedeuten, oder $R_1$ und $R_2$ bzw. $R_3$ und $R_4$ zusammen einen zweiwertigen aliphatischen oder cycloaliphatischen Kohlenwasserstoffrest, vorzugsweise einen Tetramethylen- oder Pentamethylenrest, bilden.

Vorzugsweise bedeuten in der allgemeinen Formel $R_1$,$R_2$,$R_3$ und $R_4$ Wasserstoff oder niedere Alkylreste mit 1-4 C-Atomen und R einen Alkylenrest mit 1-4 C-Atomen.

Weitere Polyepoxidharze sind zugänglich durch Kondensationsreaktionen von Epichlorhydrin mit primären und/oder sekundären Aminen, Hydroxyl- und/oder Carboxylgruppen enthaltenden Polyestern, Hydroxylgruppen aufweisenden Phenol-Formaldehyd-Kondensaten oder auch Polyätherpolyolen. Die Härtung dieser Polyepoxidharze erfolgt in an sich bekannter Weise mit Aminen bzw. Polyaminen oder Amiden bzw. Polyamiden oder mit Polycarbonsäuren, wobei unter diesem Begriff z. B. auch freie Carboxylgruppen enthaltende Polyester fallen sollen, sowie auch mit Mercapto- oder phenolische Hydroxylgruppen aufweisenden Verbindungen.

Selbstverständlich können allen diesen Lacksystemen zur Erzielung spezieller Eigenschaften auch andere Harze wie z. B. Ketonharze, Nitrocellulose, PVC-Mischpolymerisate, Cellulose-acetobutyrate usw. zugemischt werden. Auch andere in der Lackiertechnik übliche Hilfsmittel wie Verlaufshilfsstoffe, Pigmente, Füllstoffe und weitere an sich bekannte Additive können erfindungsgemäß mitverwendet werden.

Die erfindungsgemäßen Beschichtungsmassen können bis zu 90 Gew.-%, vorzugsweise bis zu 60 Gew.-%, eines organischen Lösungsmittels enthalten. Beispiele für solche Lösungsmittel sind Toluol, Xylol, aromatenarme bzw. aromatenfreie Kohlenwasserstofffraktionen, Äthylacetat, Butylacetat, Äthylenglykolmonomethylätheracetat, Äthylenglykolmonoäthylätheracetat, Aceton, Methyläthylketon, Cyclohexanon sowie Mischungen dieser Verbindungen.

Die erfindungsgemäßen Beschichtungsmassen eignen sich für die Lackierung bzw. Beschichtung beliebiger Unterlagen wie z. B. von Metallen wie Aluminium oder Stahl, von Asbestzement, Leder, Textilien, Gummi, Papier, Glas, Stein und den verschiedenartigsten Kunststoffen. Besonders geeignet sind sie für die Lackierung von Holz und Metall.

Bevorzugt werden die erfindungsgemäßen Lack- und Beschichtungssysteme maschinell verarbeitet. Man bedient sich dabei der an sich bekannten Auftragstechniken wie z. B. Spritzen, Sprühen, Tauchen, Rollen und Gießen. Die Schichtdicken liegen im allgemeinen zwischen 1 und 1 000 μm, vorzugsweise zwischen 4 und 200 μm.

Nach dem Auftragen des Lacks bzw. der Beschichtung wird mit Licht der Wellenlänge 250 bis 500 nm belichtet. Die Bestrahlungsdauer beträgt im allgemeinen 0,1 bis 300 Sekunden, vorzugsweise 1 bis 80 Sekunden, je nach der Dicke der aufgetragenen Schicht und der Leistung des Strahlers. Unter den Einfluß des kurzwelligen Lichts zerfällt, wie schon erläutert, das im Lack enthaltene erfindungsgemäße Ammoniumsalz unter Decarboxylierung. Das freiwerdende Amin wirkt dann als Katalysator für die Härtungsreaktion. Die erfindungsgemäßen Ammoniumsalze stellen oftmals auch unter dem Einfluß von Hitze tert. Amine freisetzende Verbindungen dar. Sie eignen sich daher prinzipiell auch zur Herstellung von Hitze-aktivierbaren Beschichtungsmassen auf Basis von Polyurethan- oder Polyepoxidharzvorprodukten.

Vorzugsweise wird erfindungsgemäß etwa bei Raumtemperatur (ca. 10-30 °C) beschichtet.

Die erfindungsgemäßen Beschichtungsmassen haben bei Raumtemperatur eine überraschend große

Lagerbeständigkeit, d. h. eine lange Verarbeitbarkeit; bei der Bestrahlung mit kurzwelligem Licht gelieren sie jedoch außerordentlich rasch und härten danach schnell aus — überraschenderweise auch dann, wenn es sich um keine Klarlacke sondern um pigmentierte Systeme handelt.

Ein besonderer Vorteil der erfindungsgemäßen Initiatoren ist, daß sie zu keiner Verfärbung des Lackes bei der Belichtung führen.

Die folgenden Beispiele dienen zur Erläuterung der Erfindung. Wenn nicht anders vermerkt, sind Mengenangaben als Gewichtsteile bzw. Gewichtsprozente zu verstehen.

## Beispiele

### Herstellung der erfindungsgemäßen Ammoniumsalze

Zu einer Lösung von 0,1 Mol der Carbonsäure in Aceton wird eine Lösung von 0,1 Mol tert. Amin* in Aceton zugetropft. Man erhält die Ammoniumsalze der allgemeinen Formel

$$A—(O)_m—(CH_2)_n—COO^{(-)}\quad Kat^{(+)}$$

meist in kristalliner Form.

*DMBA = Dimethylbenzylamin
DBU = Diaza-bicycloundecen

| Beispiel | A | m | n | | Amin | Schmp/° C |
|---|---|---|---|---|---|---|
| 1 | 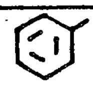 | 0 | 1 | | DMBA | Öl |
| 2 | 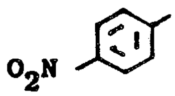 | 0 | 1 | | DMBA | 57 - 61 |
| 3 | 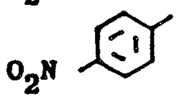 | 0 | 1 | | DBU | 71 - 74 |
| 4 | 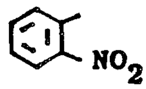 | 0 | 1 | | DBU | 76 - 82 |
| 5 | 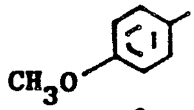 | 0 | 1 | | DMBA | 34 - 38 |
| 6 | 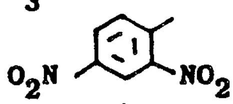 | 1 | 1 | | DMBA | Öl |
| 7 | 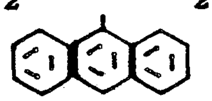 | 0 | 0 | | DMBA | 134 - 140 |
| 8 | 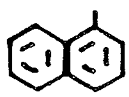 | 0 | 1 | | DMBA | Öl |
| 9 | 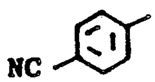 | 1 | 1 | | DMBA | Öl |
| 10 | 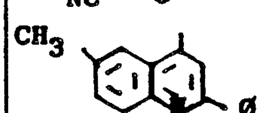 | 0 | 0 | | DMBA | Öl |
| 11 | 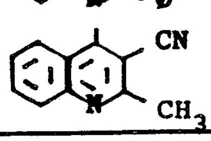 | 0 | 0 | | DMBA | Öl |

Beispiel 12

Lacke bestehend aus
264 Teilen eines pigmentierten Polyols,
100 Teilen einer 75 %igen Lösung eines biuretgruppenhaltigen aliphatischen Polyisocyanats (Umsetzungsprodukt aus 3 Mol Hexamethylendiisocyanat und 1 Mol Wasser) in Ethylenglykolmonoethyletheracetat/Xylol (1 : 1),
7 Teilen Initiator,

wurden in einer Schichtdicke von 15 μ auf Glasplatten aufgetragen und in 4 cm Abstand von einer UV-Lampe des Intensitätsmaximum bei ca. 300-320 nm 60 Sekunden lang bestrahlt, 1 Tag gelagert und auf ihre Härte untersucht. Die Ergebnisse sind in der folgenden Tabelle zusammengestellt ;
(Pendelhärte nach König = DIN 53 157)
Das pigmentierte Polyol besteht aus

100 Teilen eines Rutil-Titandioxidpigments und
164 Teilen eines Polyesters (8 % OH-Gruppen ; Säurezahl 4 ; aus 3 Mol Phthalsäureanhydrid, 0,05 Mol Maleinsäureanhydrid und 3,5 Mol Trimethylolpropan, 61 %ig gelöst in Ethylenglykolmonoethyletheracetat).

| Initiator | Pendelhärte (sec.) |
|---|---|
| keiner | 90 |
| aus Beispiel 10 | 145 |
| aus Beispiel 8 | 150 |
| aus Beispiel 9 | 120 |

**Ansprüche**

1. Ammoniumsalze, ausgewählt aus der Gruppe bestehend aus
a) Verbindungen der Formel

$$A—(O)_m—(CH_2)_n—COO^{(-)}\ Kat^{(+)}$$

in welcher
m und n gleich oder verschieden sind und für 0 oder 1 stehen, mit der Einschränkung, daß im Falle n = 0, m ebenfalls für 0 steht,
A für einen gegebenenfalls Cyano-, Nitro-, Fluor-, Chlor-, Trifluormethyl-, ($C_1$-$C_4$-Alkoxy)-carbonyl- oder ($C_1$-$C_4$-alkyl)-carbonyl-substituierten Phenyl-, 1-Naphthyl- oder Anthracyl-Rest steht oder einen gegebenenfalls Methyl-, Phenyl- oder Cyano-Substituenten und Stickstoff als Heteroatom aufweisenden heterocyclischen Rest mit 4-10 Kohlenstoffatomen steht, mit der Einschränkung, daß im Falle von m + n = 0 A einen, gegebenenfalls einen der genannten Substituenten aufweisenden Anthracyl-Rest oder einen gegebenenfalls einen der genannten Substituenten aufweisenden heterocyclischen Rest der genannten Art bedeutet und
$Kat^{(+)}$ für ein Kation steht, wie es durch Anlagerung eines Protons an ein gegebenenfalls Hydroxyl-, Mercapto-, Ether-, Thioether-, Amid-, Ester- oder Halogen-Gruppen aufweisendes, die Isocyanat-Polyadditionsreaktion beschleunigendes tertiäres Amin des Molekulargewichtsbereichs 59-200 erhalten wird,
b) Salzen der m-Methoxyphenylessigsäure und der oben bei der Definition von $Kat^{(+)}$ genannten Amine und
c) dem Dimethylbenzylamin-Salz der p-Methoxyphenylessigsäure.

2. Verfahren zur Herstellung von tertiären Aminen, dadurch gekennzeichnet, daß man Ammoniumsalze gemäß Anspruch 1 mit Licht einer Wellenlänge zwischen 250 und 500 nm bestrahlt.

3. Beschichtungsmasse auf Basis von unter dem katalytischen Einfluß von tertiären Aminen aushärtenden Polyurethan- oder Epoxyharzvorprodukten, dadurch gekennzeichnet, daß sie 0,1-10 Gew.-%, bezogen auf Feststoffgehalt, eines Ammoniumsalzes gemäß Anspruch 1 enthalten.

**Claims**

1. Ammonium salts, selected from the group consisting of

a) compounds of the formula

$$A—(O)_m—(CH_2)_n—COO^{(-)} \quad cat^{(+)}$$

in which

m and n are identical or different and represent 0 or 1 provided that, when n = 0, m also represents 0,

A represents a phenyl, 1-naphthyl or anthracyl group optionally substituted by cyano, nitro, fluoro, chloro, trifluoromethyl, ($C_1$-$C_4$-alkoxy)-carbonyl or ($C_1$-$C_4$-alkyl)-carbonyl or a heterocyclic group having 4-10 carbon atoms and containing nitrogen as hetero-atom and optionally methyl, phenyl or cyano as substituents, provided that when m + n = 0, A denotes an anthracyl group optionally containing one of the above-mentioned substituents or a heterocyclic group of the type mentioned above optionally containing one of the above-mentioned substituents and

$cat^{(+)}$ represents a cation as obtained by the addition of a proton to a tertiary amine which is of the molecular weight range of 59-200, optionally contains hydroxyl, mercapto, ether, thioether, amide, ester or halogen groups and which accelerates the isocyanate polyaddition reaction,

b) salts of m-methoxyphenyl acetic acid and the amines mentioned above under the definition of $cat^{(+)}$ and

c) the dimethylbenzylamine salt of p-methoxyphenyl acetic acid.

2. Process for the preparation of tertiary amines, characterised in that ammonium salts according to Claim 1 are irradiated with light having a wavelength of between 250 and 500 nm.

3. Coating composition based on polyurethane or epoxy resin precursors which harden under the catalytic influence of tertiary amines, characterised in that they contain 0.1-10% by weight, based on the solids content, of an ammonium salt according to Claim 1.

**Revendications**

1. Sels d'ammonium, choisis dans l'ensemble constitué par :

a) des composés de formule

$$A—(O)_m—(CH)_n—COO^{(-)} \quad Kat^{(+)}$$

dans laquelle

m et n sont identiques ou différents et valent 0 ou 1, avec la limitation que, si n est nul, m est également nul ;

A représente un reste phényle, 1-naphtyle ou anthracyle éventuellement substitué par un groupe cyano, nitro, un atome de fluor ou de chlore, un groupe trifluorométhyle, alcoxycarbonyle (1 à 4 atomes de carbone dans le groupe alcoxy) ou alkylcarbonyle (1 à 4 atomes de carbone dans le groupe alkyle), ou un reste hétérocyclique comportant 4 à 10 atomes de carbone, éventuellement substitué par un groupe méthyle, phényle ou cyano et présentant de l'azote comme hétéroatome, avec la limitation qu'au cas où (m + n) = 0, A représente un reste anthracyle présentant éventuellement l'un des substituants cités ou un reste hétérocyclique de la nature citée présentant l'un des substituants cités, et

$Kat^{(+)}$ représente un cation, tel qu'on l'obtient par fixation d'un proton sur une amine tertiaire présentant éventuellement un groupe hydroxyle, mercapto, éther, thioéther, amide, ester ou halogéno, qui accélère la réaction de polyaddition des isocyanates et dont le poids moléculaire se situe entre 59 et 200,

b) des sels de l'acide m-méthoxyphénylacétique et des amines citées ci-dessus lors de la définition de $Kat^{(+)}$, et

c) le sel de diméthylbenzylamine de l'acide p-méthoxyphényl-acétique.

2. Procédé pour produire des amines tertiaires, caractérisé en ce qu'on soumet des sels d'ammonium selon la revendication 1 à une irradiation par de la lumière dont la longueur d'onde se situe entre 250 et 500 nm.

3. Compositions d'enduction à base d'un précurseur de résines de polyuréthanne ou de résines époxydes pouvant durcir sous l'influence catalytique d'amines tertiaires, caractérisées en ce qu'elles contiennent 0,1 à 10 % en poids, par rapport à l'extrait sec, d'un sel d'ammonium selon la revendication 1.